# EUROPÄISCHE PATENTANMELDUNG

(11) **EP 0 986 991 A1**
(43) Veröffentlichungstag der Anmeldung: **22.03.2000**
(21) Anmeldenummer: 99117467.3
(22) Anmeldetag: 09.09.1999
(51) Int. Cl.: A61B 19/00

(54) **Kalibriervorrichtung**

(30) Priorität: 18.09.1998 DE 19842798
(71) Anmelder: Howmedica Leibinger GmbH & Co KG, 79111 Freiburg (DE)
(72) Erfinder: Moctezuma, José-Luis, 79104 Freiburg (DE)
(74) Vertreter: von Hellfeld, Axel, Dr. Dipl.-Phys.

(57) **Zusammenfassung**

Eine Kalibiriervorrichtung zum Erfassen der relativen Lage erstens der Achse des Schafts (Wirksachse) und zweitens des vorderen Geräteendes (Wirkpunkt bzw. Wirklinie) eines chirurgischen Geräts bezüglich einer an dem hinteren Teil des Geräts angebrachten Referenz-Positionsbestimmungseinrichtung 42 weist auf:
- zwei Klemmeinrichtungsträger mit je einer Klemmeinrichtung, wobei die Klemmeinrichtungen in einem Klemmzustand den Schaft des Geräts in eine eindeutige Klemmstellung bringen;
- eine Auflageplatte 12, auf der das vordere Geräteende in dem Klemmzustand aufliegt; und
- eine Referenz-Positionsbestimmungseinrichtung 42,
   wobei die Wirkachse in der Klemmstellung im wesentlichen senkrecht auf der Auflageplatte 12 steht und die Auflageplatte und die Referenz-Positionsbestimmungseinrichtung 42 fest miteinander verbunden sind.

## Beschreibung

Die vorliegende Erfindung bezieht sich auf eine Kalibriervorrichtung zum Erfassen der relativen Lage erstens der Achse des Schafts (Wirkachse) und zweitens des vorderen Geräteendes (Wirkpunkt, Wirklinie oder Wirkfläche) eines chirurgischen Geräts bezüglich einer am Gerät angebrachten Abstrahleinrichtung.

Moderne Bildgebungsverfahren wie Röntgentomographie und Kernspinresonanz sind heute ein wichtiges Hilfsmittel in der Chirurgie. Besonders hilfreich ist es, wenn bei operativen Eingriffen auf einem Bildschirm auch die verwendeten chirurgischen Geräte zusammen mit dem Patientenbild gezeigt werden. Hierzu muß die Lage der chirurgischen Geräte relativ zum Patienten während der Operation ständig gemessen werden.

Eine herkömmliche Methode ist dabei die Verwendung eines stereotaktischen Rahmens. Der stereotaktische Rahmen wird fest an dem Patienten angebracht und an dem Rahmen werden die chirurgischen Geräte befestigt, so daß die relative Lage bezüglich des Patienten einfach erfaßt werden kann. Eine Beschreibung stereotaktischer Rahmen findet sich in dem Prospekt "Stereotactic Treatment Planning" der Firma Howmedica Leibinger GmbH aus dem Jahre 1996. Ein Überblick über verschiedene Systeme findet sich in dem Artikel "Neuronavigation: A-ten year review" von Hans F. Reinhardt in dem Buch "Computer-integrated surgery" von R. H. Taylor, S. Lavallée; G. C. Burdea, R. Mösges; Cambridge/Massachusetts, London/England: The MIT Press (1996), Seite 329ff.

Die Anbringung eines stereotaktischen Rahmens an dem Patienten ist jedoch sehr aufwendig und für den Patienten unangenehm.

Bei moderneren Systemen wird deshalb die Lage eines chirurgischen Geräts mittels Infrarot-Strahlung gemessen (sog. "Navigator"). Dabei werden die Lage des Patienten und die Lage des chirurgischen Geräts getrennt erfaßt und zueinander in Beziehung gesetzt. Ein solches System ist beispielsweise in dem Prospekt "Experience new perspectives - with the OPM® Neuro 200 System", Fa. Carl Zeiss, D-73446 Oberkochen, gezeigt.

Die Lage des Patienten wird wie folgt vermessen: zunächst werden dem Patienten z.B. vier Schrauben eingesetzt, die mit Markierungskugeln versehen sind, die ein Kontrastmittel enthalten. Bei einer CT-Aufnahme sind diese Markierungskugeln zu sehen und können somit als Referenzpunkt dienen. Bei der Operation werden dann die Markierungskugeln entfernt, und es wird ortsgenau ein sogenannter Navigationspointer in eine verbleibende Mulde eingesetzt. Der Navigationspointer weist Infrarotleuchtdioden auf, deren Strahlung von einer z.B. über dem Operationstisch befindlichen CCD-Kamera erfaßt wird. Da mehrere Dioden gleichzeitig Strahlung aussenden, kann das System die Lage der Spitze des Navigationspointers und damit die Lage der Mulde berechnen. Wird dieses Verfahren bei allen vier Schrauben durchgeführt, so sind vier Punkte des Patienten eindeutig räumlich zugeordnet, wobei das System bereits aus drei Punkten die räumliche Lage des Patienten errechnen kann und der vierte Punkt als Kontrollpunkt fungiert.

In ähnlicher Weise wird die Lage des chirurgischen Geräts mit Infrarot-Strahlung ermittelt. An dem hinteren Teil des chirurgischen Geräts (also z.B. hinter dem Griff) wird eine Abstrahleinrichtung mit Leuchtdioden angebracht, deren Lage im Raum anhand der von der CCD-Kamera gelieferten Daten berechnet werden kann. Wenn das Computersystem die relative Lage der Gerätespitze bezüglich der Abstrahleinrichtung kennt, kann es die Lage der Gerätespitze im Raum (oder eines anderen Wirkpunktes) ebenfalls berechnen, und das Gerät kann auf dem Computerbildschirm zusammen mit dem CT-Bild dargestellt werden. Da die Abstrahleinrichtung fest mit dem chirurgischen Gerät verbunden ist, braucht die relative Lage der Spitze des Geräts zur Abstrahleinrichtung nur einmal ermittelt werden, und die entsprechenden Daten werden in das Computersystem eingegeben. Eine solche Kalibrierung ist bisher jedoch sehr aufwendig, da sie nicht vom Chirurgen selber vorgenommen werden kann.

Es wäre wünschenswert, daß der Chirurg die unterschiedlichsten chirurgischen Geräte vor oder bei einer Operation mit einer Abstrahleinrichtung fest verbinden kann und die Kalibrierung in kurzer Zeit selbst vornehmen kann. Bisher gibt es kein Verfahren für eine solche Kalibrierung, weil chirurgische Geräte die unterschiedlichsten Formen haben. Insbesondere haben nicht alle chirurgischen Geräte eine Gerätespitze (Wirkpunkt). Das Geräteende kann beispielsweise wie bei einem Meißel auch eine Kante (Wirklinie) sein oder eine Löffelform haben. Bei einem Rohr hat das Geräteende eine Wirkfläche. Eine Schwierigkeit der Ermittlung des Geräteendes besteht auch darin, daß die Achse des Schafts des Geräts, also die Wirkachse, die beispielsweise eine Drehachse sein kann, gegenüber dem hinteren Teil des Geräts weggebogen ist. Ein solches chirurgisches Gerät mit geknickter Form ist besonders schwer zu kalibrieren. Es wäre daher wünschenswert, wenn bei der Kalibrierung auch die Lage der Wirkachse ermittelt werden könnte.

Die EP 0 832 610 A2 beschreibt eine Kalibriervorrichtung zum Erfassen der relativen Lage sowohl der Achse eines Schaftes als auch des Endes eines chirurgischen Gerätes bezüglich einer in diesem angebrachten Abstrahleinrichtung. Dort wird der Abstand der Wirkfläche des Gerätes von einer Referenzfläche derart bestimmt, daß mit der Spitze des Gerätes die Referenzfläche berührt wird.

Es ist daher Aufgabe der Erfindung, eine Kalibriervorrichtung zum Erfassen der relativen Lage erstens der Achse des Schafts (Wirkachse) und zweitens des vorderen Geräteendes (Wirkpunkt, Wirklinie oder Wirkfläche) eines chirurgischen Geräts bezüglich einer am hinteren Teil des Geräts angebrachten Abstrahleinrichtung bereitzustellen, die einfach und schnell zu bedienen ist.

Die erfindungsgemäße Kalibriervorrichtung ist im Patentanspruch gekennzeichnet.

Bevorzugt weist die Kalibriervorrichtung auf: zwei Klemmeinrichtungsträger mit je einer Klemmeinrichtung, wobei die Kleinmeinrichtungen in einem Klemmzustand den Schaft des Geräts in eine in bezug auf die Kalibriervorrichtung eindeutige Klemmstellung bringen; eine Auflageplatte, auf der das vordere Geräteende in dem Klemmzustand aufliegt; und eine Referenz-Abstrahleinrichtung, wobei die Wirkachse des Geräts in der Klemmstellung im wesentlichen senkrecht auf der Auflageplatte steht und die Auflegeplatte und die Referenz-Abstrahleinrichtung der Kalibriervorrichtung fest miteinander verbunden sind.

Die erfindungsgemäße Kalibriervorrichtung hat den Vorteil, daß sie vom Chirurgen vor Ort selbst bedient werden kann. Er muß lediglich eine Abstrahleinrichtung an dem Gerät anbringen und dann das Gerät in der Kalibriervorrichtung in die Klemmstellung bringen, d.h., daß das vordere Geräteende auf der Auflageplatte aufliegt und die Klemmeinrichtungen den Schaft des Gerätes klemmen, so daß die Achse des Schafts senkrecht auf der Auflageplatte steht, und zwar kolinear mit einer durch die Klemmeinrichtungen definierten Achse der Kalibriervorrichtung. Die CCD-Kamera ermittelt nun sowohl die Lage der Abstrahleinrichtung als auch die Lage der Referenz-Abstrahleinrichtung der Kalibriervorrichtung und kann somit die relative Lage der Achse des Schafts und des Geräteendes bezüglich der Abstrahleinrichtung des Geräts erfassen. Die Lage des Geräteendes wird dabei insoweit ermittelt, als die Ebene errechnet wird, in der das Geräteende liegt.

Vorzugsweise ist mindestens ein Klemmeinrichtungsträger in der Richtung senkrecht zur Auflageplatte verschiebbar. Dadurch kann das Kalibriersystem besser an verschiedene chirurgische Geräte mit unterschiedlich langem Schaft angepaßt werden.

Vorzugsweise ist die Auflageplatte auf einer Basisplatte angeordnet, und in den Ecken eines Vierecks auf der Basisplatte sind vier senkrecht zur Auflageplatte verlaufende Säulen angebracht, wobei jeder Klemmeinrichtungsträger an mindestens einer Säule befestigt ist. Die Verwendung einer Basisplatte hat den Vorteil, daß das gesamte System stabilisiert wird. Diesem Zweck dienen auch die Säulen.

Vorzugsweise sind die Klemmeinrichtungsträger jeweils als Platte ausgebildet, die am Rand vier Führungslöcher zur Führung entlang der Säulen sowie in der Mitte eine Durchlaßöffnung für das chirurgische Gerät aufweist. Hierdurch wird die Lage der Klemmeinrichtungsträger stabilisiert, was zur Präzision der Kalibriervorrichtung beiträgt.

Bevorzugt weisen die Platten Führungshülsen zur äußeren Führung entlang der Säulen auf, und am Ende der Säulen ist ein Anschlag vorgesehen, so daß die Platten nicht über die Säulen aus dem System herausgeschoben werden können.

Der besseren Befestigung der Platten dienen Arretierungsschrauben, mittels derer sie in einer bestimmten Stellung an der Säule festgehalten werden.

Die Klemmeinrichtungen weisen in einer vorteilhaften Ausführungsform jeweils eine Durchlaßöffnung für das chirurgische Gerät und mindestens drei Greifbacken auf, die die Öffnung in einer Durchlaßstellung zumindest teilweise freigeben und vorzugsweise in einer Schließstellung völlig schließen. In der Durchlaßstellung kann das chirurgische Gerät in die Kalibriervorrichtung eingeführt werden. Dann werden die drei Greifbacken in die Klemmstellung gebracht, die eine Stellung ist, die zwischen der Durchlaßstellung und der Schließstellung liegt. Die mit dem chirurgischen Gerät in Eingriff kommenden Flächen der Greifbak ken haben gleichen radialen Abstand von der oben genannten Achse der Kalibriervorrichtung; sie definieren also diese Achse.

Vorteilhaft weisen die Greifbacken Stifte auf, und die Klemmeinrichtungen weisen jeweils eine Kulissenscheibe mit Kulissenführungen für jeden Stift der Greifbacken in der Klemmeinrichtung auf. Durch einfaches Drehen der Kulissenscheibe, beispielsweise mittels eines Dreharms, werden die Greifbacken bewegt und von der Durchlaßstellung in die Klemmstellung oder die Schließstellung und umgekehrt gebracht.

Vorzugsweise umfaßt die Referenz-Abstrahleinrichtung zwei Träger mit Abstrahlern, die parallel zu den Säulen fest an der Basisplatte angebracht sind. Auch dies trägt zur Stabilisierung des Systems und damit auch zu einer erhöhten Präzision der Kalibrierung bei.

Vorteilhaft weist jeder Träger mindestens zwei Abstrahler auf, und die Abstrahler sind LED-Infrarotdioden. Durch die Verwendung von insgesamt mindestens vier LEDs kann die Lage der Kalibriervorrichtung im dreidimensionalen Raum aus den Meßwerten für drei LEDs exakt bestimmt werden, und die vierte LED dient der Kontrolle der Messung und der Berechnung.

Wünschenswert ist es, nicht nur die Ebene zu bestimmen, in der die Wirklinie, also die Kante eines Meißels, bzw. die Wirkfläche, also die von einem Rohr umschlossene Fläche, liegt, sondern auch, wie breit die Wirklinie ist bzw. wie groß die Wirkfläche ist. Zu diesem Zweck kann an der Kalibriervorrichtung eine L-förmige Meßkante angebracht werden, was unten näher erläutert wird.

Vorteilhaft ist zur Überprüfung der bei der Kalibrierung gewonnenen Daten ein Formkörper, bevorzugt eine Kugel, fest mit der Kalibriervorrichtung verbunden.

Bevorzugte Ausführungsformen werden anhand der Zeichnungen beschrieben, wobei:
- Fig. 1: eine perspektivische Ansicht einer Kalibriervorrichtung gemäß der Erfindung von vorne zeigt;
- Fig. 2: eine Ansicht der Kalibriervorrichtung gemäß Fig. 1 von hinten zeigt;
- Fig. 3: die Draufsicht auf die Kalibriervorrichtung von Fig. 1 zeigt;
- Fig. 4: eine perspektivische Ansicht einer alternativen Ausführungsform einer Kalibriervorrichtung zeigt;
- Fig. 5: schematisch die Ermittlung der Länge der Wirklinie eines Meißels zeigt; und
- Fig. 6: schematisch die Ermittlung des Radius eines Rohres zeigt.

Auf einer dicken, schweren Basisplatte 10 ist eine Auflegeplatte 12 angeordnet. In den Ecken eines Vierecks auf der Basisplatte sind vier senkrecht zur Auflageplatte und damit auch senkrecht zur Basis verlaufende Säulen 14a, 14b, 14c, 14d angebracht. Auf den Säulen 14a-d sind gleitend eine obere Platte 16 und eine untere Platte 18 bewegbar. Die beiden Platten 16 und 18 sind baugleich und nur umgedreht in der Kalibriervorrichtung angeordnet. Aus diesem Grunde werden Bauteile der Platten 16 und 18 im folgenden nur an einer Platte der beiden mit Bezugszahlen versehen.

Jede Platte weist vier Führungslöcher 20 zur Führung entlang der Säulen auf sowie Führungshülsen 22, die die Führung verbessern. Am Ende der Säulen ist jeweils ein Anschlag 24 vorgesehen, so daß die Platten nicht, eventuell versehentlich, aus der Kalibriervorrichtung entnommen werden können. Zur Befestigung der Platten sind Arretierungsschrauben 26a, 26b, 26c und 26d vorgesehen, wobei die obere Platte 16 von den Arretierungsschrauben 26a und 26c gehalten wird, und die untere von den Arretierungsschrauben 26b und 26d gehalten wird.

Die Platten 16 und 18 haben jeweils in der Mitte eine Durchlaßöffnung 28 für das chirurgische Gerät.

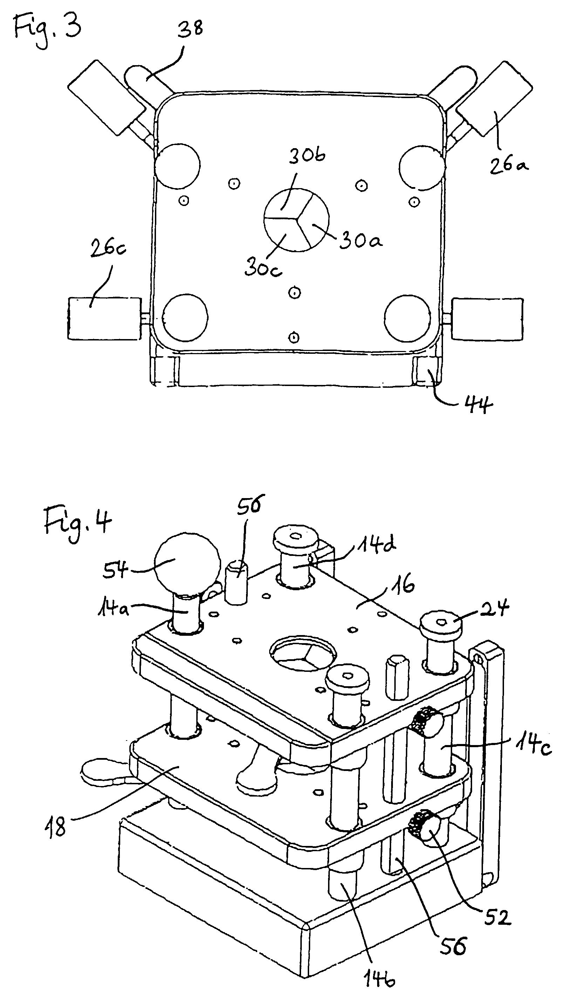

Ein chirurgisches Gerät wird in einer Klemmeinrichtung festgeklemmt, wobei die eigentliche Klemmeinrichtung aus drei Greifbacken 30a, 30b, 30c besteht, die die Öffnung 28 in einer Durchlaßstellung zumindest teilweise freigeben und in einer Schließstellung, wie sie beispielsweise in Fig. 3 gezeigt ist, völlig schließen. Die Greifbacken 30a-30c sind jeweils mit Stiften 32 versehen, die jeweils in einer Kulissenführung 34 in einer Kulissenscheibe 36 geführt werden. Die Kulissenscheibe 36 wird durch Drehen an einem Dreharm 38 betätigt. Sie sind durch Schrauben 40 jeweils an den Platten befestigt.

Um die Lage der Kalibriervorrichtung durch eine CCD-Kamera erfassen zu können, ist an der Kalibriervorrichtung eine U-förmige Referenz-Abstrahleinrichtung 42 mit zwei Trägern 44 vorgesehen, die fest mit der Basisplatte 10 verbunden ist, wobei die beiden Träger 44 parallel zu den Säulen 14a-d verlaufen. Jeder Träger 44 weist zwei LED-Infrarotdioden 46 als Abstrahler auf.

Die Bauteile der Kalibriervorrichtung bestehen nahezu ausschließlich aus Metall und sind hochpräzise gearbeitet. So ist beispielsweise die Auflageplatte 12 feinpoliert, die Greifbacken 30a-c haben eine hochpräzise Schließgenauigkeit, und die einzelnen Bauteile sind genau justiert miteinander verbunden. Für die Justierung der Referenz-Abstrahleinrichtung an der Basisplatte 10 dient beispielsweise ein Justierstift 48. Für den eigentlichen Halt sorgen Schrauben 50.

Soll nun die Kalibrierung für ein bestimmtes chirurgisches Gerät vorgenommen werden, muß zunächst an dem Gerät eine Abstrahleinrichtung fest angebracht werden. Durch geeignetes Drehen an den Dreharmen 38 werden die Greifbacken 30a-c der beiden Platten 16 und 18 jeweils in eine Durchlaßstellung gebracht. Das chirurgische Gerät kann dann durch die Öffnungen 28 geführt werden, bis es mit seinem vorderen Geräteende auf der Auflageplatte 12 zu liegen kommt. Entsprechend der Länge des Schafts können nun die Platten 16 und 18 so verstellt werden, daß der Abstand zwischen beiden maximal ist. Der Mindestabstand sollte 2 cm betragen. Dieser kann auch durch den Einbau eines Abstandsglieds zwingend vorgegeben werden. Ein solches Abstandsglied könnte beispielsweise eine ähnliche Form haben wie die Führungshülse 22 und wäre entsprechend auf der Unterseite der Platte 16 oder der Oberseite der Platte 18 anzubringen. Bei der Bewegung der Platten werden zunächst die Arretierungsschrauben aufgedreht, danach wird die Platte verschoben, und anschließend werden die Arretierungs-schrauben wieder festgedreht.

Befinden sich die obere Platte 16 und die untere Platte 18 in einer der Form des Schafts des chirurgischen Geräts entsprechenden günstigen Lage, so werden die Dreharme 38 betätigt und damit das chirurgische Gerät festgeklemmt. Durch die Form der Greifbacken ist gewährleistet, daß der Schaft des Geräts in eine eindeutige Klemmstellung gelangt, bei der die Wirkachse, also die Achse des Schafts, im wesentlichen senkrecht auf der Auflageplatte 12 steht. Nun vergleicht die Computersoftware das Signal, das die CCD-Kamera von der an dem Gerät angebrachten Abstrahleinrichtung erhält, mit der Referenz-Abstrahleinrichtung 42 der Kalibriervorrichtung. Auf diese Weise kann die Software sowohl die Lage der Ebene, in der sich das vordere Geräteende (Wirkpunkt bzw. Wirklinie) befindet, bezüglich der Lage der Abstrahleinrichtung berechnen als auch die Lage der Achse des Schafts, der Wirkachse. Dies ist insbesondere auch bei gekrümmten Geräten, bei denen der hintere Teil des Geräts von der Wirkachse wegknickt, möglich. Insgesamt ist eine Kalibrierung von etwa 95 % aller üblichen chirurgischen Geräte mit der Kalibriervorrichtung möglich.

Die Kalibrierdaten werden dann der Operationssoftware zugeführt, und durch Erfassen der Lage der Abstrahleinrichtung kann die Operationssoftware dann auf die Lage der Wirkachse und des vorderen Geräteendes rückschließen.

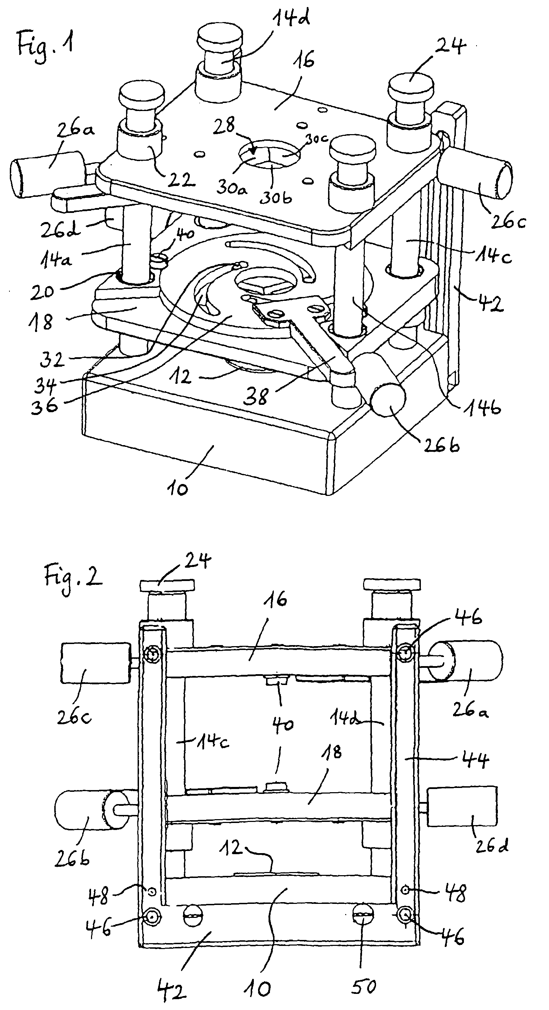

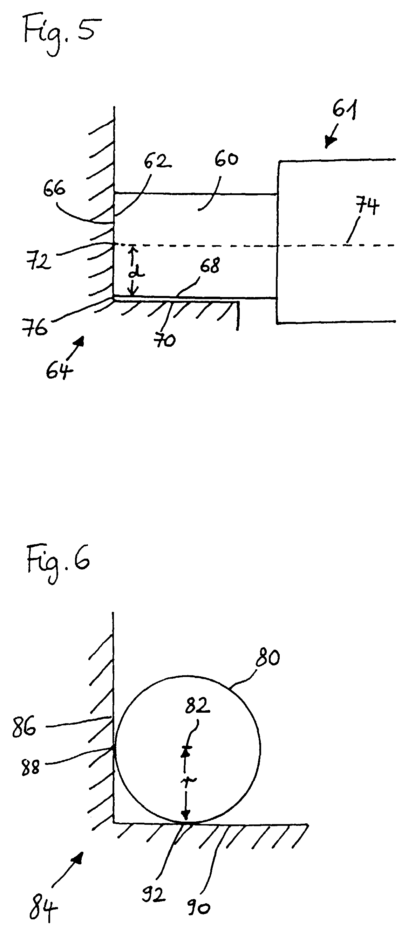

Bei der in Fig. 4 gezeigten alternativen Ausführungsform sind zusätzlich zu den Säulen 14a-14d zwei Führungsstäbe 56 vorgesehen, an denen die Platten 16 und 18 mittels Arretierungsschrauben 52 befestigt werden. Durch diese symmetrische Anordnung der Arretierungsschrauben 52 wird gegenüber der in den Fig. 1 bis 3 gezeigten Anordnung der Arretierungsschrauben 26a-d eine erhöhte Stabilität erzielt.

Die alternative Ausführungsform weist außerdem an der Säule 14a anstelle des Anschlags 24 einen Formkörper 54 auf, der beim gezeigten Ausführungsbeispiel kugelförmig ist. Mittels dieses Formkörpers können die bei der Kalibrierung gewonnenen Daten überprüft werden. Bei eingeschalteter Anzeigeeinrichtung wird der Formkörper dabei mit dem vorderen Geräteende abgetastet. Die Computersoftware vergleicht nun die ihr bekannte Lage und Form des Formkörpers, also der Kugel 54, mit der Lage, die sich bei Berechnung anhand der Kalibrierungsdaten ergibt. Auf diese Weise können letztere überprüft werden.

Die Kalibrierstation, wie sie oben beschrieben wurde, liefert Informationen über die genaue Lage der Wirkachse eines chirurgischen Geräts bezüglich der Abstrahleinrichtung und über die Ebene, in der das Geräteende liegt. Weitere Information über das Geräteende liegen jedoch nicht vor. Insbesondere bei Geräten, deren Geräteende eine bekannte Form hat, wäre es wünschenswert, Informationen auch über die Ausmaße des Geräteendes zu gewinnen.

Beispielsweise wäre es nützlich, Informationen über die Breite der Wirklinie eines Meißels bzw. den Durchmesser eines Rohres zu erfassen.

Zu diesem Zweck kann an der Kalibriervorrichtung ebenfalls ein geeigneter geometrischer Körper angebracht werden. Der einfachste Körper dieser Art ist eine L-förmige Meßkante, die fest mit der Kalibriervorrichtung verbunden ist. Sie kann beispielsweise an der oberen Platte 16 angebracht sein. Durch die feste Verbindung mit der Kalibriervorrichtung kann die genaue Lage und der Verlauf der Kante aufgrund der von der Referenzabstrahleinrichtung abgegebenen Infrarot-Strahlung ermittelt werden. In Fig. 5 ist gezeigt, wie das Vorderteil 60 eines Meißels 61 in eine Kante 64 eingeführt wird, und zwar stößt das Geräteende, die Wirklinie 62, auf die Fläche 66 der L-förmigen Kante 64, und mit der Seite 68 berührt das vordere Teil 60 des Meißels die Fläche 70 der L-förmigen Kante 64. (Nur aus zeichnerischen Gründen ist eine kleine Lücke zwischen den Flächen 68 und 70 eingezeichnet).

Aufgrund der bei der zuvor erfolgten Kalibrierung bekannten Daten kann nun der Punkt 72 berechnet werden, an dem die Wirkachse 74 die Fläche 66 berührt. Der Abstand d zwischen diesem Punkt 72 und der Ecke 76 der L-förmigen Kante 64 entspricht genau der halben Breite der Wirklinie des Meißels 61. Ausgehend von einem symmetrischen Meißel kann die Software also die Breite der Wirklinie berechnen.

In analoger Weise erfolgt die Messung des Radius und damit die Vermessung der Wirkfläche bei einem chirurgischen Gerät mit rohrförmigem Vorderteil. Dies wird schematisch anhand der Fig. 6 gezeigt. Im Unterschied zur Fig. 5 zeigt in Fig. 6 die Wirkachse des Geräts senkrecht aus der Zeichnungsebene heraus. In Fig. 6 ist der kreisförmige Umfang 80 des vorderen Geräteendes zu sehen. Die Wirkachse läuft durch den Punkt 82. Das Gerät wird senkrecht von oben in die L-förmige Kante 84 eingeführt, so daß es die Seite 86 der L-förmigen Kante 84 am Punkt 88 und die Seite 90 der L-förmigen Kante am Punkt 92 berührt. Der Abstand r zwischen dem Punkt 82 und dem Punkt 92 kann nun von der Software berechnet werden, die ja zunächst den Verlauf der Seite 90 der Kante kennt. Während der Messung wird dann Infrarot-Strahlung von der Abstrahleinrichtung an dem chirurgischen Gerät ausgesandt, und die Software kann den Punkt 82 ermitteln und somit auch den Abstand r berechnen. Alternativ oder gleichzeitig zur Kontrolle kann auch der Abstand zwischen dem Punkt 82 und dem Punkt 88 berechnet werden.

Bei den vorstehend beschriebenen Ausführungsbeispielen der Erfindung dient eine Abstrahleinrichtung 42 als Referenz-Positionsbestimmungseinrichtung. Die Abstrahleinrichtung arbeitet mit LEDs. Es ist aber auch möglich, als Referenz-Positionsbestimmungseinrichtung andere Instrumente vorzusehen und andere physikalische Wirkprinzipien, wie mechanische, elektromechanische, akustische oder magnetische Wirkprinzipien, um eine Ortung (Positionsbestimmung) vorzunehmen.

Zahlreiche Abwandlungen der beschriebenen Kalibriervorrichtung sind möglich. Beispielsweise kann die Referenz-Positionsbestimmungseinrichtung bzw. die Referenz-Positionsbestimmungseinrichtungen auch an anderer Stelle an der Kalibriervorrichtung angebracht werden. Auch ist es möglich, anstelle der Greifbacken eine Federkonstruktion zu verwenden oder eine Konstruktion wie bei Bohrfuttern. Anstelle der Platten 16 und 18 könnten auch andere Klemmeinrichtungsträger, beispielsweise ein stabiles Gitter, verwendet werden. Als Formkörper kann anstelle einer Kugel ein Quader oder eine Pyramide verwendet werden.

## Patentansprüche

1. Kalibriervorrichtung zum Erfassen der relativen Lage erstens der Achse des Schafts (Wirkachse) und zweitens des vorderen Geräteendes (Wirkpunkt, Wirklinie oder Wirkfläche) eines chirurgischen Geräts bezüglich einer am Gerät angebrachten Referenz-Positionsbestimmungseinrichtung (42), gekennzeichnet durch:
zumindest eine Klemmeinrichtung zum Einklemmen des Geräts in eine in bezug auf die Kalibriervorrichtung eindeutige Klemmstellung,
und eine Auflageplatte (12), auf der das vordere Geräteende im Klemmzustand aufliegt;
und eine Referenz-Positionsbestimmungseinrichtung (42), wobei die Wirkachse in der Klemmstellung im wesentlichen senkrecht auf der Auflageplatte (12) steht und die Auflageplatte (12) und die Referenz-Positionsbestimmungseinrichtung (42) fest miteinander verbunden sind.

2. Kalibriervorrichtung nach Anspruch 1, dadurch gekennzeichnet, daß die Referenz-Positionsbestimmungseinrichtung (42) eine Abstrahleinrichtung ist.

3. Kalibriervorrichtung nach Anspruch 1, dadurch gekennzeichnet, daß die Referenz-Positionsbestimmungseinrichtung (42) mechanisch, elektromechanisch, akustisch oder magnetisch wirkt.

4. Kalibriervorrichtung nach Anspruch 1, dadurch gekennzeichnet, daß zwei Klemmeinrichtungsträger mit je einer Klemmeinrichtung vorgesehen sind, wobei die Klemmeinrichtungen in einem Klemmzustand den Schaft des Geräts in die in bezug auf die Kalibriereinrichtung eindeutige Klemmstellung bringen.

5. Kalibriervorrichtung nach Anspruch 4, dadurch gekennzeichnet, daß mindestens ein Klemmeinrichtungsträger in der Richtung senkrecht zur Auflageplatte verschiebbar ist.

6. Kalibriervorrichtung nach Anspruch 4, dadurch gekennzeichnet, daß die Auflageplatte (12) auf einer Basisplatte (10) angeordnet ist, und daß in den Ecken eines Vierecks auf der Basisplatte vier senkrecht zur Auflageplatte verlaufende Säulen (14a-d) angebracht sind, wobei jeder Klemmeinrichtungsträger an mindestens einer Säule befestigt ist.

7. Kalibriervorrichtung nach Anspruch 6, dadurch gekennzeichnet, daß die Klemmeinrichtungsträger jeweils als Platte (16, 18) ausgebildet sind, die am Rand vier Führungslöcher (22) zur Führung entlang der Säulen sowie in der Mitte eine Durchlaßöffnung (28) für das chirurgische Gerät aufweist.

8. Kalibriervorrichtung nach Anspruch 7, dadurch gekennzeichnet, daß die Platten Führungshülsen (24) zur Führung entlang der Säulen (14a-d) aufweisen, und daß am Ende der Säulen ein Anschlag (24) vorgesehen ist.

9. Kalibriervorrichtung nach Anspruch 7, dadurch gekennzeichnet, daß die Platten (16, 18) Arretierungsschrauben (26a-d) aufweisen, mittels derer sie in einer bestimmten Stellung an der Säule (14a-d) festgehalten werden.

10. Kalibriervorrichtung nach Anspruch 1, dadurch gekennzeichnet, daß die Klemmeinrichtungen jeweils eine Durchlaßöffnung (28) für das chirurgische Gerät und mindestens drei Greifbacken (30a-c) aufweisen, die die Öffnung (28) in einer Durchlaßstellung zumindest teilweise freigeben und vorzugsweise in einer Schließstellung völlig schließen.

11. Kalibriervorrichtung nach Anspruch 10, dadurch gekennzeichnet, daß die Greifbachen (30a-c) Stifte (32) aufweisen und die Klemmeinrichtungen jeweils eine Kulissenscheibe (36) mit Kulissenführungen (34) für jeden Stift (32) der Greifbacken (30a-c) in der Klemmeinrichtung aufweisen.

12. Kalibriervorrichtung nach Anspruch 11, dadurch gekennzeichnet, daß die Kulissenscheibe (36) mittels eines Dreharms (38) drehbar ist.

13. Kalibriervorrichtung nach Anspruch 1, dadurch gekennzeichnet, daß die Referenz-Positionsbestimmungseinrichtung (42) zwei Träger (44) mit Abstrahlern (46) umfaßt, die parallel zu den Säulen (14a-d) fest an der Basisplatte (10) angebracht sind.

14. Kalibriervorrichtung nach Anspruch 13, dadurch gekennzeichnet, daß jeder Träger (44) mindestens zwei Abstrahler aufweist.

15. Kalibriervorrichtung nach Anspruch 14, dadurch gekennzeichnet, daß die Abstrahler LED-Infrarotdioden (46) sind.

16. Kalibriervorrichtung nach Anspruch 1, dadurch gekennzeichnet, daß eine Meßkante fest mit ihr verbunden ist.

17. Kalibriervorrichtung nach Anspruch 1, dadurch gekennzeichnet, daß ein Formkörper fest mit ihr verbunden ist.

18. Kalibriervorrichtung nach Anspruch 17, dadurch gekennzeichnet, daß der Formkörper eine Kugel (54) ist.
